# EUROPEAN PATENT APPLICATION

(11) **EP 4 379 732 A1**
(43) Date of publication of application: **05.06.2024**
(21) Application number: 22210580.1
(22) Date of filing: 30.11.2022
(51) Int. Cl.: G16H 10/20, G16H 10/60, G06F 21/62

(54) **SYSTEM AND METHOD FOR PROVIDING MEDICAL INFORMATION**

(71) Applicant: Dedalus HealthCare GmbH, 53227 Bonn (DE)
(72) Inventor: BATSELIER, Koenraad, 9920 Lievegem (BE); EBY, Colin Aaron, London, Greater London, NW1 9DY (GB)
(74) Representative: Linsmeier, Josef

(57) **Abstract**

The invention relates to a method and a corresponding system for providing medical information from a plurality of data sources (1), in particular healthcare organizations such as hospitals and/or medical offices, to a plurality of data users (4, 10), in particular clinical researchers and/or research institutions. The system comprises: a) a plurality of first processing appliances (2) each operatively coupled to a respective data source (1), each first processing appliance (2) being configured to i) retrieve and/or receive from the respective data source (1), one or more electronic medical records (5), each electronic medical record (5) containing data associated with an individual patient, and ii) to generate, for each electronic medical record (5), a metadata record (MDR) by labeling data contained in the respective electronic medical record (5) with metadata regarding a plurality of different categories including at least one of the following patient-related categories: date or year of birth, age, location, disease, allergy, medication, observations and/or procedures a patient has undergone, and including, in the metadata record (MDR), metadata and therewith labeled data in accordance with a predefined list (whitelist) of metadata to be included, while excluding and/or obfuscating data and/or metadata in accordance with at least one predefined condition ("blocklfRare", "blocklfAge>95") for at least one of the patient-related categories of metadata and therewith labeled data; b) at least one first storage appliance (6) configured to retrieve and/or receive the metadata records (MDR) from the first processing appliances (2) and to store the retrieved and/or received metadata records (MDR); and c) at least one access control appliance (9) configured to control, in particular to grant and/or deny, access of the data users (4, 10) to the first storage appliance (9).

## Description

The present disclosure relates to a system and corresponding method for providing medical information from a plurality of data sources, in particular healthcare organizations such as hospitals and/or medical offices, to a plurality of data users, in particular clinical researchers and/or research institutions.

A typical clinical research process follows a multi-step process from an initial idea through to review and approval, including patient enrolment and data collection for clinical studies. The success of the enrolment and data collection phase is significantly influenced by the ability to find suitable patients that not only match the trial criteria but have a willingness and commitment to enroll in a multi-year research program. However, when enabling clinical researchers to effectively use healthcare data from various sources, such as healthcare providers, data privacy must be taken into account.

It is an object of present disclosure to provide a system and a method for providing medical information from a plurality of data sources, in particular a plurality of healthcare providers, to a plurality of data users, in particular a plurality of clinical researchers or research institutions, which is improved regarding data privacy, in particular as to protecting and/or anonymizing the identity of the data sources and/or their patients.

This object is achieved by a system and a method according to the independent claims. Preferred embodiments are the subject of the independent claims.

A first aspect of present disclosure relates to a system for providing medical information from a plurality of data sources, in particular healthcare organizations such as hospitals and/or medical offices, to a plurality of data users, in particular clinical researchers and/or research institutions, wherein the system comprises: a) a plurality of first processing appliances each operatively coupled to a respective data source, each first processing appliance being configured to i) retrieve and/or receive from the respective data source, one or more electronic medical records, each electronic medical record containing data associated with an individual patient, and ii) to generate, for each electronic medical record, a metadata record by labeling, also referred to as "tagging", data contained in the respective electronic medical record with metadata, also referred to as "tags" or "tags", regarding a plurality of different categories including at least one of the following patient-related categories: date or year of birth, age, location, disease, allergy, medication, observations and/or procedures a patient has undergone, and including, in the metadata record, metadata and therewith labeled data in accordance with a predefined list, also referred to as "whitelist", of metadata to be included, while excluding and/or obfuscating data and/or metadata in accordance with at least one predefined condition for at least one of the patient-related categories of metadata and therewith labeled data; b) at least one first storage appliance, also referred to as "metadata observatory (MDO)", configured to retrieve and/or receive the metadata records from the first processing appliances and to store the retrieved and/or received metadata records, and c) at least one access control appliance configured to control, in particular to grant and/or deny, access of the data users to the first storage appliance.

A second aspect of present disclosure relates to a method for providing medical information from a plurality of data sources, in particular healthcare organizations such as hospitals and/or medical offices, to a plurality of data users, in particular clinical researchers and/or research institutions, each data source being operatively coupled to a respective first processing appliance (Edge), wherein: a) each first processing appliance i) retrieves and/or receives from the respective data source, one or more electronic medical records, each electronic medical record containing data associated with an individual patient, and ii) generates, for each electronic medical record, a metadata record by labeling, also referred to as "tagging", data contained in the respective electronic medical record with metadata, also referred to as "tag" or "tags", regarding a plurality of different categories including at least one of the following patient-related categories: date or year of birth, age, location, disease, allergy, medication, observations and/or procedures a patient has undergone, and including, in the metadata record, metadata and therewith labeled data in accordance with a predefined list, also referred to as "whitelist", of metadata to be included, while excluding and/or obfuscating data and/or metadata in accordance with at least one predefined condition for at least one of the patient-related categories of metadata and therewith labeled data; b) at least one first storage appliance, also referred to as "metadata observatory (MDO)", retrieves and/or receives the metadata records from the first processing appliances and stores the retrieved and/or received metadata records, and c) at least one access control appliance controls, in particular grants and/or denies, access of the data users to the first storage appliance.

A third aspect of present disclosure relates to a computer program product causing a computer, computer system and/or distributed computing environment (e.g. a client-server system or storage and processing resources of a computer cloud) to execute the method according to the second aspect of present disclosure.

A fourth aspect of present disclosure relates to a computer, computer system and/or distributed computing environment (e.g. a client-server system or storage and processing resources of a computer cloud) comprising means for carrying out the method according to the second aspect of present disclosure.

Aspects of present disclosure are preferably based on the approach of providing, via an at least one first storage appliance or metadata observatory (MDO) and at least one access control appliance, metadata records to data users, wherein in the metadata records predefined or "white-listed" metadata and therewith labeled data associated with individual patients is included, while data and/or metadata, which is basically white-listed for being included in the metadata record but which fulfills a predefined condition, is excluded from and/or obfuscated in the metadata records. Preferably, the at least one condition is predefined for at least one patient-related category of white-listed metadata, such as date or year of birth, age, location, disease, allergy, medication, observations and/or procedures a patient has undergone, and therewith labeled data. Preferably, data labeled with the white-listed categories of metadata are usually included in a metadata record, unless data labeled with metadata fulfills a predefined condition for this metadata and therewith labeled data. In this case, this data, and optionally also the respective metadata, is excluded from the metadata record or at least obfuscated in the metadata record.

For example, a predefined condition may require that data labeled with "age" (metadata) is larger than a predefined value, for example "95". If the labeled data in a medical record is e.g. "91", the predefined condition is not fulfilled, so that the data "91" labeled with the metadata "age" is included in and/or not excluded from the metadata record. If, however, the particular data is e.g. "97", the predefined condition is fulfilled so that the data "97" labeled with the metadata "age" is not included in and/or is excluded from and/or obfuscated in the metadata record. In this way, it can be reliably avoided that, even in the absence of patient identifying data, such as name, address and/or patientID, in the metadata record, an individual patient can be re-identified "indirectly", i.e. because of his or her particularly high age.

Within the context of present disclosure, the term "predefined condition" preferably relates to and/or comprises any conditional, conditional statement or conditional expression which performs different computations or actions, such as excluding metadata and/or therewith labeled data from the metadata record, depending on whether a predefined boolean condition (e.g. data labeled with "age" is larger than "95") evaluates to true or false.

It is noted that data exclusion and/or obfuscation may not necessarily concern the same data and/or metadata for which a predefined condition is given, but also other data which are not necessarily subject to the predefined condition itself. For example, a predefined condition may require that data (e.g. "muscular dystrophy") labeled with "disease" (metadata) is contained in a predefined list of a rare diseases and that, if so, other data (e.g. "Cologne") labeled with "location" (metadata) is not included in and/or is excluded from and/or obfuscated in the metadata record, whereas the data (e.g. "muscular dystrophy") labeled with "disease" is included in and/or not excluded from the metadata record. In this way, it can be reliably avoided that, even in the absence of patient and/or data source identifying data, such as a patientID and/or sourcelD, in the metadata record, an individual patient and/or a healthcare provider can be re-identified "indirectly", i.e. due to the occurrence of a rare disease at a location. At the same time, it is ensured that medically relevant data, such as the presence of a rare disease, is not excluded from the metadata record.

As illustrated by the above examples, the system and method according to present disclosure are designed for examining metadata and therewith labeled data in view of one or more predefined conditions, and processing said data and metadata and/or other data and metadata according to process step(s) specified for the respective predefined condition, in particular by excluding and/or obfuscating specified data, if a predefined condition is fulfilled. In this way, the anonymization of the data contained in the metadata records to be provided to data users is further improved while preserving medically relevant information as far as possible.

In summary, present disclosure provides an improved system and method which not only allows for including "white-listed" metadata and therewith labeled data in metadata records provided to data users, but also for considering predefined conditions in order to specifically exclude and/or obfuscate data which are likely to enable a data user having access to the metadata records to "indirectly" re-identify data sources and/or individual patients.

In the context of present disclosure, the term "data source" preferably relates to any device, in particular a computer or computer network, associated with and/or located at a healthcare organization such as a hospital and/or medical office and configured to acquire and/or store data relating to individual patients, including personal and health-related data, as well as data on services and procedures to which the patient is/or has been subjected.

In the context of present disclosure, the term "data user" preferably relates to any device, in particular a computer or computer network, associated with and/or located at a clinical researcher and/or research institution.

In the context of present disclosure, the terms "processing appliance", "storage appliance", and "access control appliance" preferably relate to any device, in particular a processor, a memory, a computer, a computer network and/or cloud computing network, which is configured to handle, process and/or store data and/or control access to stored data as respectively specified.

In the context of present disclosure, the terms "electronic medical record (EMR)", "electronic health record (EHR)" and "electronic patient record (EPR)" are used synonymously, unless otherwise stated. Preferably, the term "electronic medical record (EMR)" relates to a patient record created by providers for specific encounters in hospitals and ambulatory environments. Preferably, an EMR or EHR relates to a systematized collection of electronically stored patient health information in a digital format. These records can be shared across different health care settings. Records can be shared through network-connected, enterprise-wide information systems or other information networks and exchanges. EMRs or EHRs may include a range of data, including demographics, medical history, medication and allergies, immunization status, laboratory test results, radiology images, vital signs, personal statistics like age and weight, and billing information.

Preferably, the one or more predefined conditions and process step(s) specified for a respective predefined condition, in particular as to excluding and/or obfuscating specified data, are preferably stored in a data storage which is operatively coupled to a first processing appliance. Preferably, one or more user interfaces are provided which enable authorized users to input and/or change the predefined conditions and process step(s) associated therewith.

Preferably, the at least one predefined condition is suitable and/or configured to characterize at least one rare or non-standard medical case. For example, the at least one predefined condition may be a condition for data labeled with metadata which is contained in a predefined list of rare or non-standard medical cases, or not contained in a predefined list of standard medical cases. This allows for reliably identifying data which are likely to allow for a re-identification of an individual patient and/or a healthcare provider due to the rare occurrence of the respective medical case yet without having patient and/or data source identifying data, such as a patientID and/or sourcelD, in the metadata records.

Preferably, each first processing appliance is configured to exclude and/or obfuscate data and/or metadata relating to the date and/or year of birth and/or age of a patient, if at least one of the following predefined conditions is fulfilled: the date or year of birth of the patient is prior to a predefined date or year of birth and/or the age of the patient exceeds a predefined age. As already explained above, this allows for reliably avoid that, even in the absence of patient identifying data, such as name, address and/or patientID, in the metadata record, an individual patient can be re-identified because of his or her particularly early date or year of birth and/or particularly high age.

Preferably, each first processing appliance is configured to exclude and/or obfuscate data and/or metadata relating to the location regarding a patient and/or a respective data source, if at least one of the following predefined conditions is fulfilled: the disease of the patient is included in at least one predefined list of diseases, in particular rare diseases; the allergy of the patient is included in at least one predefined list of allergies, in particular rare allergies; the medication of the patient is included in at least one predefined list of medications, in particular rare medications; and/or the procedure the patient has undergone is included in at least one predefined list of procedures, in particular rare procedures. In this way, it can be reliably avoided that, even in the absence of patient and/or data source identifying data such as a patientID and/or sourcelD in the metadata records, an individual patient and/or a healthcare provider can be re-identified due to the rare occurrence of the respective medical case.

Preferably, at least one of the first processing appliances is configured to, in particular prior to generating the metadata record: determine, in the data associated with an individual patient, unstructured text information; determine, in the unstructured text information, at least one patient identifier, such as the name and/or patient ID of the patient, by which the individual patient can be identified; and remove the at least one patient identifier from the unstructured text information. Preferably, the at least one of the first processing appliances is configured to, in particular prior to generating the metadata record, convert the unstructured text information into structured medical information, in particular by performing natural language processing (NLP) of the unstructured text information. For example, unstructured medical and/or clinical text may comprise physician's notes, letters, discharge summaries, test results, and case notes. In this way, unstructured text information can be converted into structured medical information including both data, which is extracted from the text information, and metadata by which the extracted data is labeled, but no data by which the individual patient can be identified.

For example, an NLP process preferably comprises the following steps:
- Input of unstructured text
- Named entity recognition by recognizing relevant clinical terms in the unstructured text, and
- Named entity normalization by assigning recognized terms with a standard medical terminology (sample output).
This will be further illustrated by examples given in the figure description below.

Preferably, at least one of the first processing appliances is configured to, in particular after removing the at least one patient identifier from the unstructured text information and prior to generating the metadata record, convert the data contained in the electronic medical records into the Fast Healthcare Interoperability Resources (FHIR) standard describing a data format for exchanging the electronic medical records. In this way, disparate data sources providing different formats and terminology codes can be simplified to help the data users more easily explore the metadata records without having to deal with native data formats.

Preferably, at least one of the first processing appliances is configured to: determine, in multiple electronic medical records and/or metadata records each containing data associated with the same individual patient, at least one patient identifier (patient ID) by which the individual patient can be identified; replace, in each of the multiple electronic medical records and/or metadata records, the patient identifier by a unique collection identifier (collection ID) which allows to identify the multiple electronic medical records and/or metadata records as relating to the same patient; and include the collection identifier in the metadata records before the metadata records are retrieved and/or received by the at least one first storage appliance, wherein, after the collection identifier is included in the metadata records and/or after the metadata records are retrieved and/or received by the at least one first storage appliance, the collection identifier is no longer accessible to the at least one of the first processing appliances. In this way, it is ensured that data users can recognize if multiple metadata records relate to the same patient. Further, any link between the patient identifier and the collection identifier is cut, so that a re-identification of the individual patient by the collection identifier is impossible.

Preferably, at least one of the first processing appliances is configured to: identify, in the data labeled with the metadata, one or more encounter dates regarding an encounter of an individual patient in the healthcare organization; perform a date shifting operation on the encounter dates by, in particular randomly, shifting the encounter dates to obtain shifted encounter dates, while preferably preserving the sequence of the encounter dates and/or the duration of a period of time defined by the encounter dates; and including, in the metadata record, the shifted encounter dates instead of the one or more encounter dates. For example, a date shifting rule is to replace the day in the month of all date shift required fields with a randomly generated integer between 1 and 28. The year and month will remain the same. For example, if a patient has a hospital visit date of 05-Oct-2020, this will be shifted to any date between 01-0ct-2020 to 28-Oct-2020.

Preferably, at least one of the first processing appliances is configured to: identify, in the data labeled with the metadata, a date of birth of an individual patient; perform a date reducing operation on the date of birth by reducing the date of birth to the year of birth; and including, in the metadata record, the year of birth instead of the date of birth of the individual patient.

Alternatively, at least one of the first processing appliances is configured to: identify, in the data labeled with the metadata, a date of birth of an individual patient; perform a date shifting operation on the date of birth by, in particular randomly, shifting the date of birth to obtain a shifted date of birth; and including, in the metadata record, the shifted date of birth instead of the date of birth of the individual patient. For example, a date shifting rule is to replace the day in the month of the date of birth field with a randomly generated integer between 1 and 28. The year and month will remain the same. For example, if a patient has a date of birth of 05-Oct-1955, this will be shifted to any date between 01-Oct-1955 to 28-Oct-1955.

Preferably, at least one of the first processing appliances is configured to: identify, in the data labeled with the metadata, one or more location data regarding a location of an individual patient and/or of the healthcare organization; perform a data obfuscating operation on the location data by obfuscating the location data to country level to obtain obfuscated location data; and including, in the metadata record, the obfuscated location data instead of the one or more location data.

At least one of the operations described above, or a combination thereof, makes a re-identification of the individual patient even more difficult.

Preferably, at least one of the first processing appliances is configured to: identify, in the data labeled with the metadata, one or more identifiers by which at least one of the following can be identified: the data source (EdgeSourceID), in particular a healthcare organization, a visit (VisitID) or an encounter (EncounterlD) of an individual patient in the healthcare organization; perform a hashing operation on the identifiers, in particular by SHA-512 hashing, particular irreversible hashing, to obtain hashed identifiers; and including, in the metadata record, the hashed identifiers instead of the identifiers. In this way, while data users cannot track back the data source, visit or encounter, respectively, by means of the hashed identifiers included in the metadata records, authorized institutions such as the service providers having access to the original identifiers (prior to being hashed) can do so, if required. It is noted that, in some cases, "Visit ID" and "Encounter ID" may be used interchangeably depending on the system of record. Preferably, they both refer to a unique identifier created when a patient visits a healthcare facility for an inpatient, outpatient or emergency visit which may lead to a condition being asserted, an observation being made, a procedure performed, or medication prescribed.

Preferably, the system comprises at least one second processing appliance configured to, before the access control appliance grants access of the data users to the metadata record stored in the first storage appliance, encrypt at least one of the hashed identifiers, in particular a hashed data source identifier (EdgeSourceID), included in the metadata record stored in the at least one first storage appliance (MDO). In this way, it is ensured that the correlation or link between the data provider (source), on the one hand, and the metadata record, on the other hand, is protected particularly well from the data users. At the same time, it allows for an attribution of a metadata record to a data provider (source) by an authorized institution such as a data service provider being able to decrypt the hashed identifier.

Further advantages, features and examples of the present disclosure will be apparent from the following description of figures showing:
- Fig. 1: a schematic representation of an example of a system for providing medical information from at least one data source to at least one data user;
- Fig. 2: a schematic representation of another example of a system for providing medical information from at least one data source to at least one data user;
- Fig. 3: a schematic representation of an example for handling a patient identifier (patient ID) in a system for providing medical information from at least one data source to at least one data user;
- Fig. 4: examples of different whitelists regarding patient, condition and encounter;
- Fig. 5: further examples of different whitelists regarding medication statement, observation and procedure; and
- Fig. 6: examples for illustrating an NLP process.

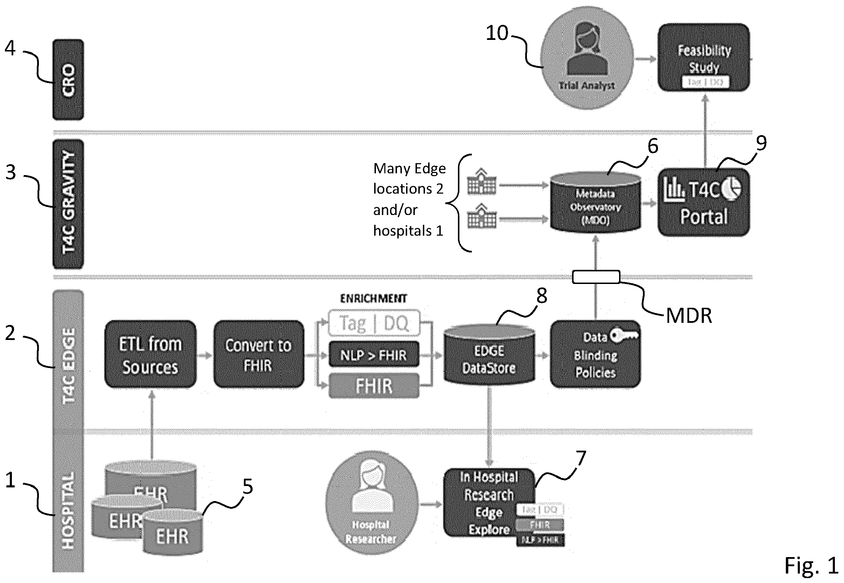

Fig. 1 shows a schematic representation of an example of a system for providing medical information from at least one data source 1, for example a hospital, to at least one data user 4, for example a clinical research organization (CRO).

The system comprises at least one first processing appliance 2 which is operatively coupled to the data source 1 and configured to retrieve and/or receive from the data source 1 one or more electronic medical records 5, each electronic medical record 5 containing data associated with an individual patient.

Preferably, the at least one first processing appliance 2 is also referred to as "T4C Edge" or "Edge" and refers to data and services under direct control of the data source 1, i.e. the hospital. "T4C" is an abbreviation of the term "Trials4Care" relating to the use of an application programming interface (API) which enables clinical researchers to effectively use healthcare data from various sources to improve clinical research outcomes.

As will be explained in more detail further below and exemplarily shown in present example, Edge 2 is configured to perform at least one of the following:
- data extraction, transformation and loading (ETL), in particular extracting data from various sources 1 and/or electronic medical records 5;
- converting native and/or proprietary source and/or electronic medical record formats to FHIR standard format;
- performing natural language processing (NLP) for unstructured data;
- adding clinical and/or analytical "tags" (also referred to as "labeling" or "tagging") such as #diabetes, #hypertension etc. to group condition, observation, encounter, medication statements and procedure concept codes; and
- anonymizing and/or blinding and/or obfuscating patient identifiable data.

As a result, metadata records MDR are obtained that can be shared, via a preferably central first storage appliance 6 which is also referred to as "metadata observatory" (MDO), with the data users 4.

Optionally, at the level of the hospital 1 and/or Edge 2, an explore appliance 7 may be provided which allows in-hospital researchers to access to an Edge datastore 8, in which metadata records prior to data anonymizing and/or blinding and/or obfuscating are stored, for local research.

Preferably, the first processing appliance 2 and/or the explore appliance 7 reside within a local datacenter and/or the hospital's cloud subscription.

Preferably, the first storage appliance 6 is part of a central instance 3, also referred to as "T4C Gravity", provided between a plurality of data sources 1 and respective first processing appliances 2, on the one hand, and a plurality of data users 4, on the other hand. T4C Gravity 3 is preferably configured as a centralized research data network that aggregates metadata into the metadata observatory 6 from disparate data sources 1 and/or Edge 2 services and provides it, preferably through a secure marketplace and at least one access control appliance 9 which is also referred to as "T4C portal", to research organizations 4 which can perform feasibility studies with the obscured data.

Preferably, researchers 10 can raise requests to collaborate further with the care provider(s) 1 through a secure channel (not shown) without knowing the identity of the data provider 1 and their patients.

Preferably, the first processing appliance 2 is configured to generate, for each electronic medical record 5, a metadata record MDR by labeling, also referred to as "tagging", data contained in the respective electronic medical record 5 with metadata, also referred to as "tags", regarding a plurality of different categories including at least one of the following patient-related categories: date or year of birth, age, location, disease, allergy, medication, observations and/or procedures a patient has undergone, and including, in the metadata record MDR, metadata and therewith labeled data in accordance with a predefined list, also referred to as "whitelist", of metadata to be included.

Prior to forwarding the respective metadata record MDR to the first storage appliance 6, data and/or metadata are deleted and/or excluded from and/or obfuscated in the metadata record MDR in accordance with at least one predefined condition for at least one of the patient-related categories of metadata and therewith labeled data. In other words, metadata and therewith labeled data is examined with respect to one or more predefined conditions and, if a predefined condition is fulfilled, data and metadata, which is specified for the respective predefined condition, is deleted and/or excluded from and/or obfuscated in the metadata record MDR.

Preferably, the at least one predefined condition relates to at least one rare and/or non-standard medical case, for example when the age of a patient exceeds a predefined age, the patient has a rare disease or allergy, the patient receives a rare medication and/or the patient has undergone a rare medical procedure. In this way it can be reliably avoided that, even in the absence of data source 1 and/or patient identifying data in the metadata record, such as name, address, patient ID and/or source ID, the data source 1 and/or individual patient can be re-identified "indirectly" due to the occurrence of a rare case at a location. This allows for an improved anonymization of the data contained in the metadata records MDR, but without significantly reducing the medically relevant content of the metadata records MDR.

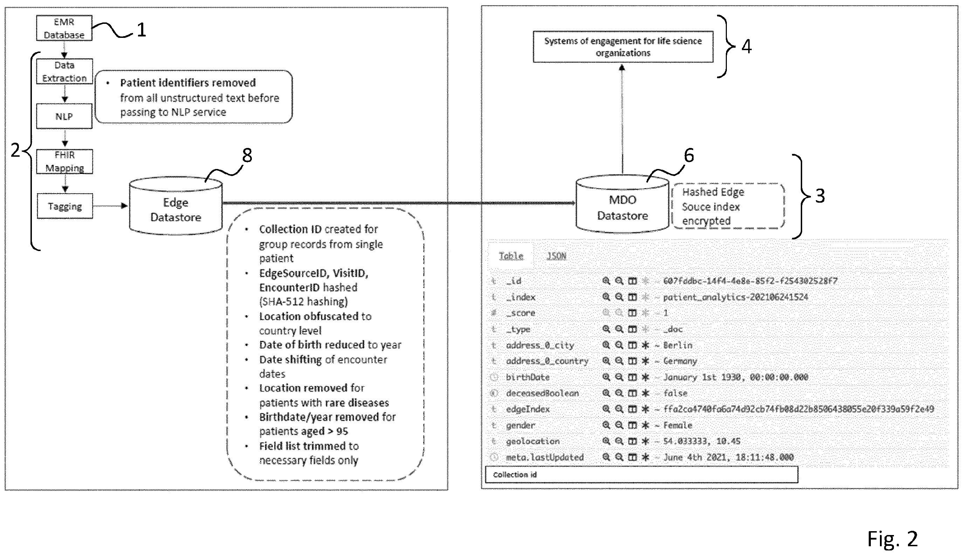

Fig. 2 shows another schematic representation of an example of a system for providing medical information from at least one data source 1 to at least one data user 4.

The system is configured to extract both structured and unstructured data, for example as csv files, from various electronic medical record (EMR) systems 1, which can be located in different countries, and stores them as FHIR resources within Edge datastore 8, preferably in JSON file format.

Preferably, the unstructured data are passed through natural language processing (NLP) services to extract meaningful metadata and then, together with the structured data, converted into FHIR resources and stored on Edge datastore 8. Preferably, before passing to NLP, patient identifiers are removed from all unstructured text contained in the received electronic medical records 1.

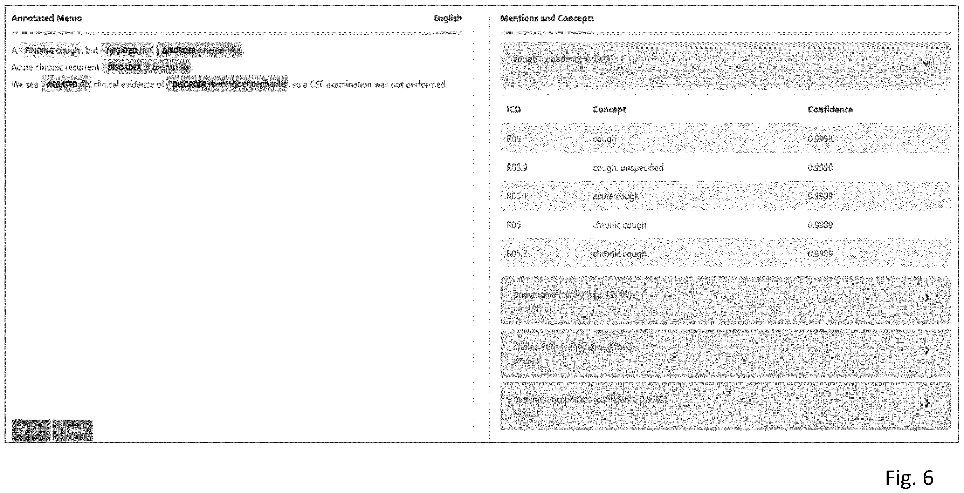

An NLP process preferably comprises the following steps:
- Input of unstructured text, for example "*A cough, but not pneumonia. Acute chronic recurrent cholecystitis. We see no clinical evidence of meningoencephalitis, so a CSF examination was not performed.*"
- Named entity recognition by recognizing relevant clinical terms in the unstructured text, as exemplarily shown as "Annotated Memo" in Fig. 6 (left).
- Named entity normalization by assigning recognized terms with a standard medical terminology (sample output), as exemplarily shown as "Mentions and Concepts" in Fig. 6 (right)

The FHIR data then goes through a tagging process to assign accurate and meaningful tags making the data usable for analytics. Preferably, for each FHIR record, a tagged copy is created for at least one of the following resources: conditions, encounters, patients, procedures, observations, and medication statements. Preferably, all tagging operations are overseen by qualified clinicians previously instructed by healthcare providers.

Preferably, during tagging and/or after having been stored in the Edge datastore 8 the data goes through a series of anonymization and/or encryption steps to, preferably completely, obfuscate patient identifiable data, so that only the analytical tags also referred to as "metadata records", are moved to first storage appliance 6, also referred to as "Meta Data Observatory (MDO)". Preferably, all patient identifiable data is removed from the records stored in the Edge data store 8 prior to replicating them from Edge 2 to the MDO 6 at T4C Gravity 3. For example, during tagging, advanced age may be obfuscated by replacing age data for example with "age greater than 95". It is further preferred that, during tagging, the location of patients with rare diseases may be obfuscated.

From the MDO 6, the anonymized analytical tags (metadata records) can then be queried by the data users 4 such as life science organizations for suitable candidate identification.

In present example, similarly to the example of the system shown in Fig. 1, data extraction, NLP, FHIR mapping, tagging, anonymization and/or obfuscation are performed by a first processing appliance 2, also referred to as "T4C Edge", and the first storage appliance 6 is part of a central instance 3, also referred to as "T4C Gravity". As to that, the above explanations regarding Fig. 1 apply accordingly to Fig. 2.

Preferably, records from a single patient are grouped using a Collection ID so that the Patient ID does not need to be moved to T4C Gravity 3 and hence enable a further anonymization of the metadata records.

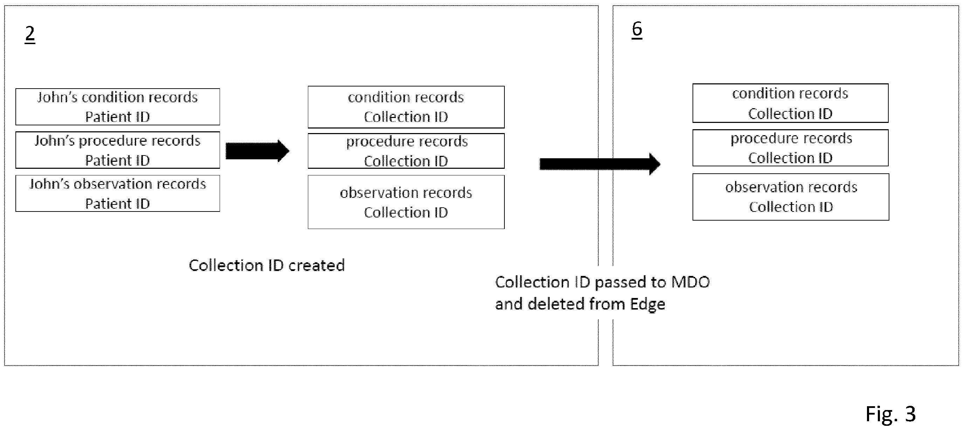

This is illustrated in Fig. 3, which shows a schematic representation of in total three different records, also referred to as "group of records", regarding conditions, procedures and observation for the same patient, who is identifiable by a unique patient ID contained in each of the records. As schematically indicated in the figure, a unique collection ID is created which replaces the patient ID in each of the records for this patient. When passing the group of records, in particular the group of metadata records, including the collection ID (instead of the patient ID) to the MDO 6, the collection ID is deleted from Edge 2. Preferably, creating the collection ID and replacing the patient ID by the collection ID only takes place in the processing appliance/Edge 2, wherein the collection ID is not stored in Edge 2 or only temporarily stored, for example in a volatile or dynamic core memory or RAM of the processing appliance/Edge 2. In this way it is ensured that the collection ID can never re-used by Edge 2 for the purpose of re-identifying an individual patient based on a collection ID. The collection ID's only function is to be able to relate a patient's records together, without actually mapping those records to an individual or a source record.

As indicated in the example shown in Fig. 2, further measures for improved and/or advanced anonymization comprise at least one of the following: the location of a patient is obfuscated to country level; the date of birth of a patient is reduced to year; date shifting operations are performed to obfuscate date of birth and/or encounter dates; birth date/or birth year is removed for patients with an age higher than 95; the location for patients with rare diseases (optionally also rare allergies, rare medications and/or rare medical procedures) is removed; the metadata field, which is also referred to as "whitelist", is trimmed to only necessary fields to minimize the data.

As further indicated in the example shown in figure 2, identifiers of the data source (EdgeSourceID), in particular a healthcare organization, a visit (VisitID) or an encounter (EncounterID) of an individual patient in the healthcare organization are hashed, in particular by SHA-512 hashing, to obtain respectively hashed identifiers which are included in the metadata record instead of the original identifiers. This reliably ensures that data users cannot track back the data source, visit and/or encounter, respectively, by means of the hashed identifiers included in the metadata records, whereas authorized institutions such as service providers having access to the original identifiers (prior to being hashed) can do so, if required.

Preferably, on the level of T4C Gravity 3, a supplemental encryption on the previously hashed data source identifier (EdgeSourceID) is added. This ensures that the correlation or link between the data provider (source), on the one hand, and the metadata record, on the other hand, is protected particularly well from the data users. At the same time, it allows for an attribution of a metadata record to a data provider (source) by an authorized institution such as a data service provider being able to decrypt the hashed data source identifier.

As already described above, the metadata records MDR include preferably only metadata or tags, which is or are preferably predefined by one or more so-called whitelists, and therewith labeled data, while some data and/or metadata is excluded (also referred to as "blocked") from and/or obfuscated in the metadata records, if at least one predefined condition for at least one category of metadata, such as year of birth, age, location, disease, allergy, medication, observations and/or procedures a patient has undergone, and therewith labeled data is fulfilled.

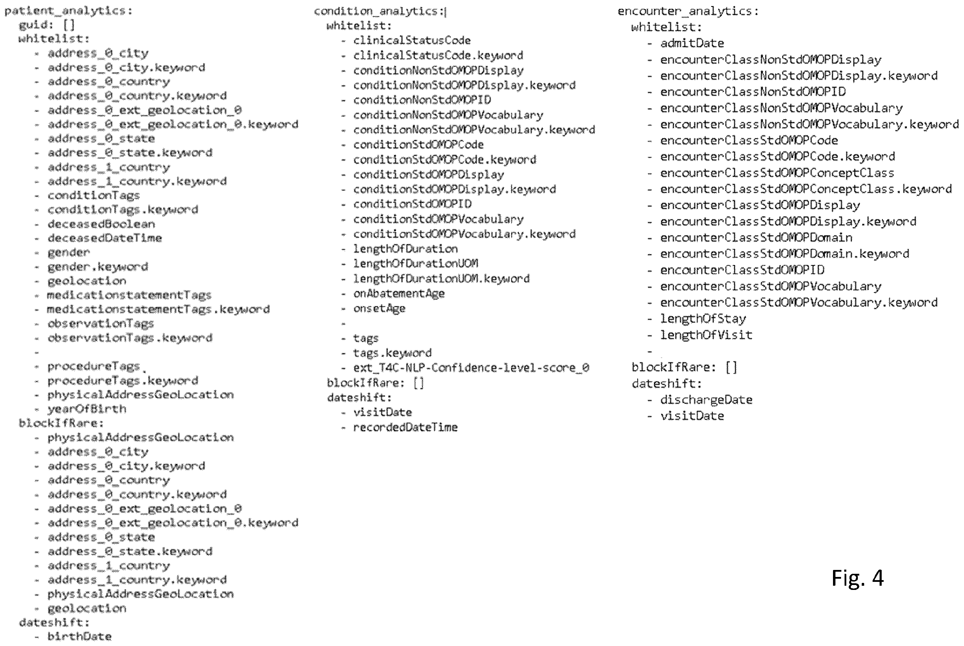

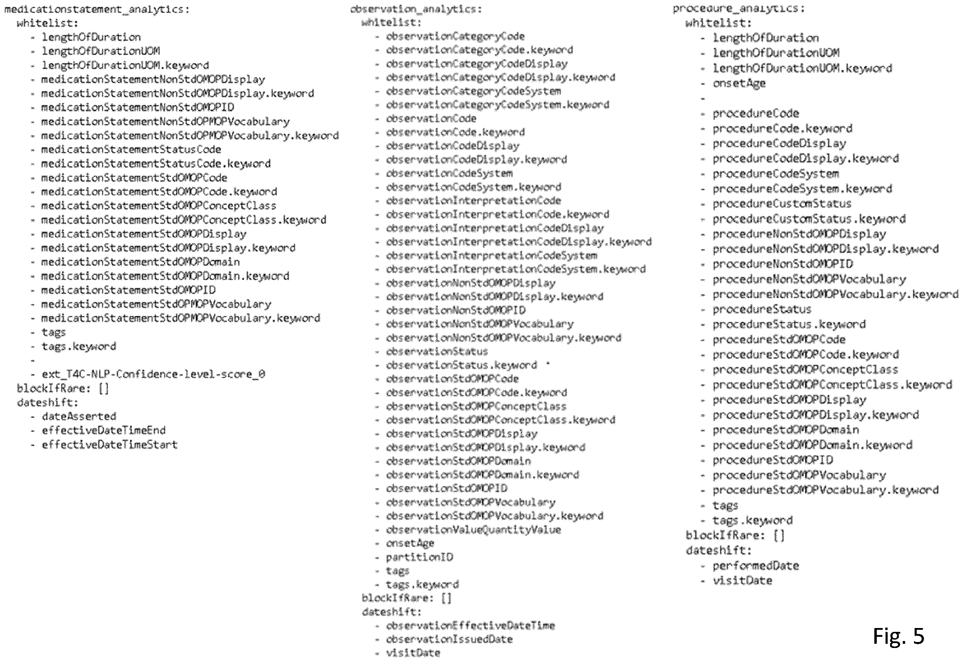

Figs 4 and 5 show examples of different whitelists regarding "patient", "condition", "encounter", "medication statement", "observation", and "procedure" and the respectively included tags such as "address", "gender" and "year of birth" in the whitelist regarding "patient", "on abatement age" and "onset age" in the whitelist regarding "condition" or "admit data", "length of stay" and "length of visit" in the whitelist regarding "encounter".

Further, as indicated by "blocklfRare" at the end of the whitelist regarding "patient" in Fig. 4, a conditional is predefined according to which tags regarding the location of the patient are blocked, i.e. excluded, from the metadata record, if the patient represents a rare medical case, for example because of a rare disease, rare allergy, rare medication and/or rare medical procedure (not indicated in present schematic representation).

Alternatively or additionally, further conditions and/or conditionals can be predefined in a similar way, for example a conditional "blocklfAge>95" (not shown) according to which the tag "year of birth" (or at least the data labeled therewith) is blocked from the metadata record, if the age of the patient exceeds 95. Another example is the conditional block of location details, if the patient is known to have a rare condition.

Preferably, patient-related categories of white-listed metadata include, but are not limited to, at least one of the following categories:. Preferably, data labeled with the white-listed categories of metadata are usually included in a metadata record, unless particular data labeled with metadata fulfills a predefined condition for this metadata and therewith labeled data. In this case, this data, and optionally also the respective metadata, is excluded from the metadata record or at least obfuscated in the metadata record.

Additionally, for each whitelist one or more tags can be specified which, before being included in the respective metadata record, undergo a date shifting operation, such as "birth date" for the whitelist "patient", "visit date" and "recorded date time" for the whitelist "condition" or "discharge date" and "visit date" for the whitelist regarding "encounter".

## Claims

1. A system for providing medical information from a plurality of data sources (1), in particular healthcare organizations such as hospitals and/or medical offices, to a plurality of data users (4, 10), in particular clinical researchers and/or research institutions, the system comprising:
a) a plurality of first processing appliances (2) each operatively coupled to a respective data source (1), each first processing appliance (2) being configured to i) retrieve and/or receive from the respective data source (1), one or more electronic medical records (5), each electronic medical record (5) containing data associated with an individual patient, and ii) to generate, for each electronic medical record (5), a metadata record (MDR) by
- labeling data contained in the respective electronic medical record (5) with metadata regarding a plurality of different categories including at least one of the following patient-related categories: date or year of birth, age, location, disease, allergy, medication, observations and/or procedures a patient has undergone, and
- including, in the metadata record (MDR), metadata and therewith labeled data in accordance with a predefined list (whitelist) of metadata to be included, while excluding and/or obfuscating data and/or metadata in accordance with at least one predefined condition ("blocklfRare", "block-IfAge>95") for at least one of the patient-related categories of metadata and therewith labeled data,
b) at least one first storage appliance (6) configured to retrieve and/or receive the metadata records (MDR) from the first processing appliances (2) and to store the retrieved and/or received metadata records (MDR), and
c) at least one access control appliance (9) configured to control, in particular to grant and/or deny, access of the data users (4, 10) to the first storage appliance (9).

2. The system according to claim 1, wherein the at least one predefined condition is configured to characterize at least one rare medical case.

3. The system according to claim 1 or 2, wherein each first processing appliance (2) is configured to exclude and/or obfuscate data and/or metadata relating to the date and/or year of birth and/or age of a patient if at least one of the following predefined conditions is fulfilled: the date or year of birth of the patient is prior to a predefined date or year of birth, and/or the age of the patient exceeds a predefined age.

4. The system according to any one of the preceding claims, wherein each first processing appliance (2) is configured to exclude and/or obfuscate data and/or metadata relating to the location of a patient if at least one of the following predefined conditions is fulfilled:
- the disease of the patient is included in at least one predefined list of diseases, in particular rare diseases,
- the allergy of the patient is included in at least one predefined list of allergies, in particular rare allergies,
- the medication of the patient is included in at least one predefined list of medications, in particular rare medications,
- the procedure the patient has undergone is included in at least one predefined list of procedures, in particular rare procedures.

5. The system according to any one of the preceding claims, wherein at least one of the first processing appliances (2) is configured to, in particular prior to generating the metadata record (MDR),
- determine, in the data associated with an individual patient, unstructured text information,
- determine, in the unstructured text information, at least one patient identifier (patient ID) by which the individual patient can be identified, and
- remove the at least one patient identifier from the unstructured text information.

6. The system according to claim 5, wherein at least one of the first processing appliances (2) is configured to, in particular prior to generating the metadata record (MDR), convert the unstructured text information into structured medical information, in particular by performing natural language processing (NLP) of the unstructured text information.

7. The system according to any one of the preceding claims, wherein at least one of the first processing appliances (2) is configured to, in particular after removing the at least one patient identifier from the unstructured text information and prior to generating the metadata record (MDR), convert the data contained in the electronic medical records (5) into the Fast Healthcare Interoperability Resources (FHIR) standard describing a data format for exchanging the electronic medical records.

8. The system according to any one of the preceding claims, wherein at least one of the first processing appliances (2) is configured to
- determine, in multiple electronic medical records (5) each containing data associated with the same individual patient, at least one patient identifier (patient ID) by which the individual patient can be identified,
- replace, in each of the multiple electronic medical records, the patient identifier by a unique collection identifier (collection ID) which allows to identify the multiple electronic medical records as relating to the same patient, and
- include the collection identifier in the metadata records (MDR) generated from the multiple electronic medical records (5) before the metadata records (MDR) are retrieved and/or received by the at least one first storage appliance (6),
wherein, after the collection identifier is included in the metadata records (MDR) and/or after the metadata records (MDR) are retrieved and/or received by the at least one first storage appliance (6), the collection identifier is no longer accessible to the at least one of the first processing appliances (2).

9. The system according to any one of the preceding claims, wherein at least one of the first processing appliances (2) is configured to
- identify, in the data labeled with the metadata, one or more encounter dates regarding an encounter of an individual patient in the healthcare organization,
- perform a date shifting operation on the encounter dates by, in particular randomly, shifting the encounter dates to obtain shifted encounter dates, while preferably preserving the sequence of the encounter dates and/or the duration of a period of time defined by the encounter dates, and
- including, in the metadata record (MDR), the shifted encounter dates instead of the one or more encounter dates.

10. The system according to any one of the preceding claims, wherein at least one of the first processing appliances (2) is configured to
- identify, in the data labeled with the metadata, a date of birth of an individual patient,
- perform a date reducing operation on the date of birth by reducing the date of birth to the year of birth, and
- including, in the metadata record (MDR), the year of birth instead of the date of birth of the individual patient.

11. The system according to any one of the preceding claims, wherein at least one of the first processing appliances (2) is configured to
- identify, in the data labeled with the metadata, one or more location data regarding a location of an individual patient and/or of the healthcare organization,
- perform a data obfuscating operation on the location data by obfuscating the location data to country level to obtain obfuscated location data, and
- including, in the metadata record (MDR), the obfuscated location data instead of the one or more location data.

12. The system according to any one of the preceding claims, wherein at least one of the first processing appliances (2) is configured to
- identify, in the data labeled with the metadata, one or more identifiers by which at least one of the following can be identified: the data source (EdgeSourceID), in particular a healthcare organization, a visit (VisitID) and/or an encounter (EncounterID) of an individual patient in the healthcare organization,
- perform a hashing operation on the identifiers, in particular by SHA-512 hashing, to obtain hashed identifiers, and
- including, in the metadata record (MDR), the hashed identifiers instead of the identifiers.

13. The system according to claim 12, further comprising at least one second processing appliance configured to, before the access control appliance (9) grants access of the data users (4, 10) to the metadata record (MDR) stored in the first storage appliance (6), encrypt at least one of the hashed identifiers, in particular a hashed encounter identifier (EncounterID), included in the metadata record (MDR) stored in the at least one first storage appliance (6).

14. A method for providing medical information from a plurality of data sources (1), in particular healthcare organizations such as hospitals and/or medical offices, to a plurality of data users (4, 10), in particular clinical researchers and/or research institutions, each data source (1) being operatively coupled to a respective first processing appliance (2), wherein:
a) each first processing appliance (2) i) retrieves and/or receives from the respective data source (1), one or more electronic medical records (5), each electronic medical record (5) containing data associated with an individual patient, and ii) generates, for each electronic medical record (5), a metadata record (MDR) by
- labeling data contained in the respective electronic medical record (5) with metadata regarding a plurality of different categories including at least one of the following patient-related categories: date or year of birth, age, location, disease, allergy, medication, observations and/or procedures a patient has undergone, and
- including, in the metadata record (MDR), metadata and therewith labeled data in accordance with a predefined list (whitelist) of metadata to be included, while excluding and/or obfuscating data and/or metadata in accordance with at least one predefined condition ("blocklfRare", "block-IfAge>95") for at least one of the patient-related categories of metadata and therewith labeled data,
b) at least one first storage appliance (6) retrieves and/or receives the metadata records (MDR) from the first processing appliances (2) and stores the retrieved and/or received metadata records (MDR), and
c) at least one access control appliance (9) controls, in particular grants and/or denies, access of the data users (4, 10) to the first storage appliance 6.

15. A computer program product causing a computer, computer system and/or distributed computing environment, in particular a client-server system or storage and processing resources of a computer cloud, to execute the method according to claim 14.
